(19)

Europäisches
Patentamt
European
Patent Office
Office européen
des brevets

(11) **EP 4 089 067 A1**

(12) **EUROPEAN PATENT APPLICATION**
published in accordance with Art. 153(4) EPC

(43) Date of publication:
**16.11.2022 Bulletin 2022/46**

(21) Application number: **20912253.0**

(22) Date of filing: **09.12.2020**

(51) International Patent Classification (IPC):
**C07C 29/76** (2006.01)     **C07C 33/26** (2006.01)

(52) Cooperative Patent Classification (CPC):
**C07C 29/76; C07C 33/26**

(86) International application number:
**PCT/JP2020/045843**

(87) International publication number:
**WO 2021/140820 (15.07.2021 Gazette 2021/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **10.01.2020   JP 2020002979**

(71) Applicant: **SUMITOMO CHEMICAL COMPANY,
LIMITED
Tokyo 103-6020 (JP)**

(72) Inventors:
• **KOIKE, Hirofumi**
  **Sodegaura-shi, Chiba 299-0246 (JP)**
• **SAWANO, Naoto**
  **SINGAPORE LTD., 100, Ayer Merbau Road
  628277 (SG)**
• **YOSHII, Masayuki**
  **Ichihara-shi, Chiba 299-0195 (JP)**

(74) Representative: **Winter, Brandl - Partnerschaft
mbB
Alois-Steinecker-Straße 22
85354 Freising (DE)**

(54)     **METHOD FOR PRODUCING BIS(2-HYDROXY-2-PROPYL)BENZENE**

(57)     An object is to provide a method for producing bis(2-hydroxy-2-propyl)benzene, the method making it possible to prevent blockage in a pipe in a step of drying bis(2-hydroxy-2-propyl)benzene and thereby enhance the production efficiency. The above object is attained by the production method including a drying step of drying aqueous bis(2-hydroxy-2-propyl)benzene under a condition in which bis(2-hydroxy-2-propyl)benzene does not sublime and which lies below a vapor pressure curve of water.

FIG. 1

EP 4 089 067 A1

## Description

Technical Field

The present invention relates to a method for producing bis(2-hydroxy-2-propyl)benzene.

Background Art

A method for producing bis(2-hydroxy-2-propyl)benzene is disclosed in, for example, Patent Literature 1, which discloses a method of simultaneously producing dihydroxybenzene and bis(2-hydroxy-2-propyl)benzene by using diisopropylbenzene as a raw material. Patent Literature 1 discloses purifying the resultant bis(2-hydroxy-2-propyl)benzene through crystallizing, filtering, and drying operations.

Citation List

[Patent Literature]

[Patent Literature 1]
Japanese Patent Application Publication, Tokukai, No. 2007-246512

Summary of Invention

Technical Problem

For the production of bis(2-hydroxy-2-propyl)benzene, a drying step of causing water to evaporate is essential because the resultant bis(2-hydroxy-2-propyl)benzene includes water irrespective of synthesis process thereof. Despite that, in producing bis(2-hydroxy-2-propyl)benzene by the method disclosed in Patent Literature 1, a blockage in a pipe of a producing device can occur in the step of drying the synthesized bis(2-hydroxy-2-propyl)benzene. Whenever a pipe becomes blocked in the drying step, it is necessary to stop the production to remove the blocking substance. This causes a reduction in the efficiency of producing bis(2-hydroxy-2-propyl) benzene.
Previously, the cause of the blockage in a pipe remained unknown.
An object of an aspect of the present invention is to provide a method for producing bis(2-hydroxy-2-propyl)benzene, the method making it possible to prevent blockage in a pipe in the step of drying bis(2-hydroxy-2-propyl)benzene and thereby enhance the production efficiency.

Solution to Problem

As a result of a diligent study to attain the above object, the inventors of the present invention made the first-ever finding that the blockage in a pipe is caused by sublimation of bis(2-hydroxy-2-propyl)benzene. Further, on the basis of such a finding, the inventors performed the drying under a condition in which bis(2-hydroxy-2-propyl)benzene does not sublime and which lies below a vapor pressure curve of water. From this, the inventors found that blockage in a pipe can be prevented and the production efficiency can be thereby enhanced, and completed the present invention. The present invention includes the following inventions [1] to [5].

[1] A method for producing bis(2-hydroxy-2-propyl)benzene including a drying step of drying aqueous bis(2-hydroxy-2-propyl)benzene containing at least water, the method including a drying step of drying the aqueous bis(2-hydroxy-2-propyl)benzene under a condition in which bis(2-hydroxy-2-propyl)benzene does not sublime and which lies below a vapor pressure curve of water.
[2] The method described in [1], in which in the drying step, the aqueous bis(2-hydroxy-2-propyl)benzene is dried at a temperature and a pressure that satisfy Expression (1) below and that lie below a vapor pressure curve of water:

$$P \geq 4 \times 10^{-21} \times T^{11} \quad (1),$$

where T is temperature (in °C) and P is pressure (in kPaA, absolute pressure).
[3] The method described in [1] or [2], in which the aqueous bis(2-hydroxy-2-propyl)benzene is at least one selected from 1,3-(2-hydroxy-2-propyl)benzene and 1,4-(2-hydroxy-2-propyl)benzene.
[4] The method described in any one of [1] to [3], in which the aqueous bis(2-hydroxy-2-propyl)benzene further contains organic solvent.

[5] The method described in [4], in which the organic solvent is at least one selected from diisopropylbenzene, methyl isobutyl ketone, phenethyl ether, diisopropyl ether, benzyl alcohol, and phenylethanol.

Advantageous Effects of Invention

The method, in accordance with an embodiment of the present invention, for producing bis(2-hydroxy-2-propyl)benzene yields an effect of preventing blockage in a pipe in a step of drying a resultant bis(2-hydroxy-2-propyl)benzene and thereby enabling enhancement of the production efficiency.

Brief Description of Drawings

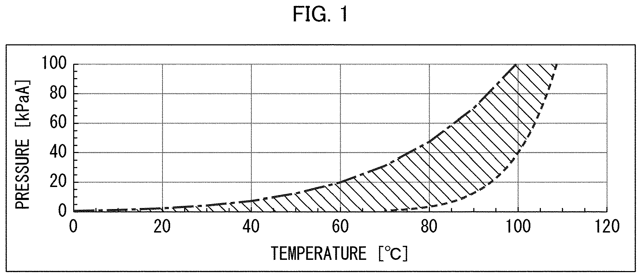

Fig. 1 is a view schematically illustrating a condition in which bis(2-hydroxy-2-propyl)benzene does not sublime and which lies below a vapor pressure curve of water.

Description of Embodiments

The following description will discuss in detail embodiments of the present invention. As used herein, the phrase "A to B" refers to "not less than A and not more than B".

A method, in accordance with an embodiment of the present invention, for producing bis(2-hydroxy-2-propyl)benzene is a method for producing bis(2-hydroxy-2-propyl)benzene including a drying step of drying aqueous bis(2-hydroxy-2-propyl)benzene containing at least water, the method including a drying step of drying the aqueous bis(2-hydroxy-2-propyl)benzene under a condition in which bis(2-hydroxy-2-propyl)benzene does not sublime and which lies below a vapor pressure curve of water.

Steps of [1.1] drying step and [1.2] other steps of the method, in accordance with an embodiment of the present invention, for producing bis(2-hydroxy-2-propyl)benzene are described in this order below.

Drying step

A method, in accordance with an embodiment of the present invention, for producing bis(2-hydroxy-2-propyl)benzene is a method for producing bis(2-hydroxy-2-propyl)benzene including a drying step of drying aqueous bis(2-hydroxy-2-propyl)benzene containing at least water, the method including a drying step of drying the aqueous bis(2-hydroxy-2-propyl)benzene under a condition in which bis(2-hydroxy-2-propyl)benzene does not sublime and which lies below a vapor pressure curve of water.

In an embodiment of the present invention, aqueous bis(2-hydroxy-2-propyl)benzene containing at least water and to be dried in the present drying step only needs to be bis(2-hydroxy-2-propyl)benzene obtained in a step of synthesizing bis(2-hydroxy-2-propyl)benzene, and can therefore be, for example, crude bis(2-hydroxy-2-propyl)benzene obtained by separating and/or purifying bis(2-hydroxy-2-propyl)benzene contained in a reaction liquid produced as a result of the synthesis. The bis(2-hydroxy-2-propyl)benzene obtained in the step of synthesizing bis(2-hydroxy-2-propyl)benzene contains, irrespective of synthesis process thereof, water such as water contained in a raw material liquid in the synthesis reaction, water in which a reducing agent is dissolved, water obtained as a by-product of a reduction reaction, or water mixed in a cleaning step, a crystallizing step, and a separating step. The method, in accordance with an embodiment of the present invention, for producing bis(2-hydroxy-2-propyl)benzene can therefore be suitably used in a bis(2-hydroxy-2-propyl)benzene production method in which any synthesis process is used.

The water content of the aqueous bis(2-hydroxy-2-propyl)benzene to be subjected to the present drying step is not limited to any particular amount, but is preferably not more than 40 mass%, more preferably not more than 20 mass%, and even more preferably 10 mass%, relative to the mass of the aqueous bis(2-hydroxy-2-propyl)benzene to be subjected to the present drying step. The water content of the aqueous bis(2-hydroxy-2-propyl)benzene to be subjected to the present drying step is preferably not more than 40 mass% because the drying duration will not be unnecessarily long. The lower limit of the water content of the aqueous bis(2-hydroxy-2-propyl)benzene is preferably 1 mass% relative to the mass of the aqueous bis(2-hydroxy-2-propyl)benzene to be subjected to the drying step.

The aqueous bis(2-hydroxy-2-propyl)benzene to be subjected to the present drying step may further contain an organic solvent. In the synthesis, separation, and purification of bis(2-hydroxy-2-propyl)benzene, an organic solvent is typically used. The aqueous bis(2-hydroxy-2-propyl)benzene to be subjected to the present drying step can therefore contain the above-described organic solvent. Examples of the organic solvent can include, but not particularly limited to, hydrocarbons, alcohols, ketones, and ethers. Among others, the organic solvent is more preferably at least one organic solvent selected from diisopropylbenzene, methyl isobutyl ketone, phenethyl ether, diisopropyl ether, benzyl alcohol, and phenylethanol.

The amount of organic solvent contained in the aqueous bis(2-hydroxy-2-propyl)benzene to be subjected to the present drying step is not limited to any particular amount, but is preferably not more than 40 mass%, more preferably not more than 20 mass%, and even more preferably not more than 10 mass%, relative to the mass of the aqueous bis(2-hydroxy-2-propyl)benzene to be subjected to the present drying step. The amount of the organic solvent contained in the aqueous bis(2-hydroxy-2-propyl)benzene to be subjected to the present drying step is preferably not more than 40 mass% because the drying duration will not be unnecessarily long. The lower limit of the amount of the organic solvent contained in the aqueous bis(2-hydroxy-2-propyl)benzene is preferably 1 mass% relative to the mass of the aqueous bis(2-hydroxy-2-propyl)benzene to be subjected to the drying step.

As used in an embodiment of the present invention, the term "drying" refers to making the water content of the bis(2-hydroxy-2-propyl)benzene after dried smaller than the water content of the aqueous bis(2-hydroxy-2-propyl)benzene before dried. The water content of the bis(2-hydroxy-2-propyl)benzene after dried is preferably less than 1 mass% relative to the mass of the bis(2-hydroxy-2-propyl)benzene after dried.

When the aqueous bis(2-hydroxy-2-propyl)benzene to be subjected to the present drying step further contains an organic solvent, the amount of the organic solvent contained in the bis(2-hydroxy-2-propyl)benzene after dried is preferably less than 1 mass% relative to the mass of the bis(2-hydroxy-2-propyl)benzene after dried.

In an embodiment of the present invention, the drying is carried out under a condition in which bis(2-hydroxy-2-propyl)benzene does not sublime and which lies below a vapor pressure curve of water. Carrying out the drying under a condition in which bis(2-hydroxy-2-propyl)benzene does not sublime prevents blockage in a pipe in the drying step and thereby enables enhancement of the production efficiency.

Here, the condition in which bis(2-hydroxy-2-propyl)benzene does not sublime means a temperature and pressure condition satisfying the following Expression (1).

$$P \geq 4 \times 10^{-21} \times T^{11} \quad (1)$$

In Expression (1), T is temperature (in °C) and P is pressure (in kPaA).

In the drying step in accordance with an embodiment of the present invention, the aqueous bis(2-hydroxy-2-propyl)benzene is dried at a temperature and a pressure that satisfy Expression (1) and that lie below the vapor pressure curve of water. Carrying out the drying at a temperature and a pressure lying below the vapor pressure curve of water efficiently removes the water contained in the aqueous bis(2-hydroxy-2-propyl)benzene.

The condition in which bis(2-hydroxy-2-propyl)benzene does not sublime and which lies below a vapor pressure curve of water is explained with reference to Fig. 1. In Fig. 1, a line indicated by a dot-dashed line is the vapor pressure curve of water and a line indicated by a dashed line is the line representing $P = 4 \times 10^{-21} \times T^{11}$. As can be seen, drying the aqueous bis(2-hydroxy-2-propyl)benzene under a condition in which benzene does not sublime and at a temperature and a pressure lying below the vapor pressure curve of water refers to drying at a pressure and a temperature in the region indicated by the hatching in Fig. 1.

In an embodiment of the present invention, drying only needs to be carried out under a condition in which bis(2-hydroxy-2-propyl)benzene does not sublime and which lies below a vapor pressure curve of water. In another embodiment of the present invention, the drying may be carried out under a condition which lies below a vapor pressure curve of the organic solvent in addition to the condition described above. The drying may be more preferably carried out under a condition which lies below a vapor pressure curve of at least one organic solvent selected from diisopropylbenzene, methyl isobutyl ketone, phenethyl ether, diisopropyl ether, benzyl alcohol, and phenylethanol in addition to the condition in which bis(2-hydroxy-2-propyl)benzene does not sublime and which lies below the vapor pressure curve of water. This makes it possible to accelerate the removal of the organic solvent in the drying.

The aqueous bis(2-hydroxy-2-propyl)benzene only needs to be at least one selected from 1,3 -(2-hydroxy-2-propyl) benzene and 1,4-(2-hydroxy-2-propyl)benzene. The aqueous bis(2-hydroxy-2-propyl)benzene is more preferably 1,3-(2-hydroxy-2-propyl)benzene.

Other steps

The method for producing bis(2-hydroxy-2-propyl)benzene in accordance with an embodiment of the present invention only needs to include the drying step, and can include, for example, a synthesizing step of synthesizing bis(2-hydroxy-2-propyl)benzene and a purifying step of purifying the resultant bis(2-hydroxy-2-propyl)benzene, which are other steps.

(Synthesizing step)

In an embodiment of the present invention, the synthesizing step is not limited to any particular step provided that the step is a step of synthesizing bis(2-hydroxy-2-propyl)benzene. Further, the synthesis may take any synthesis route.

The synthesizing step is not limited thereto, but can be, for example, a step of obtaining a reaction liquid containing bis(2-hydroxy-2-propyl)benzene by hydrogenating aromatic peroxides such as 1-(hydroperoxyisopropyl)-3-(hydroxyisopropyl)benzene in the presence of an alumina-supported transition metal catalyst.

In an embodiment of the present invention, the alumina-supported transition metal catalyst may be any catalyst that is a transition metal catalyst supported on alumina. The transition metal catalyst can be at least one selected from palladium, nickel, and platinum. In the alumina-supported transition metal catalyst, the concentration of the transition metal catalyst supported on an alumina support is typically 0.01 mass% to 20 mass% relative to the mass of the alumina support.

In an embodiment of the present invention, the reaction pressure of hydrogenation in the synthesizing step is typically 0 MPa to 10 MPa (gauge pressure). The reaction temperature in the synthesizing step is typically 20°C to 200°C and the reaction time in the synthesizing step is typically 1 minute to 300 minutes. The reaction method in the synthesizing step can be a liquid-phase fixed bed flow reaction or a stirred tank slurry reaction. Among these reactions, a stirred tank slurry reaction with the use of a powder catalyst is suitable from the viewpoint of, for example, reactivity, selectivity, and catalyst life.

(Purifying step)

In an embodiment of the present invention, the purifying step is a step of purifying bis(2-hydroxy-2-propyl)benzene contained in the reaction liquid containing bis(2-hydroxy-2-propyl)benzene obtained in the synthesizing step. The purification is not limited to any particular method, but can be, for example, a method involving crystallizing and filtering operations, a method involving a distilling operation, a method involving a cleaning operation, or a method involving an adsorption operation. In the purifying step, a concentrating operation may be carried out before the crystallizing operation. The concentration operation is an operation for partial removal of the organic solvent by distillation

The crystallizing operation is an operation of causing bis(2-hydroxy-2-propyl)benzene to be selectively deposited. For example, the reaction liquid containing bis(2-hydroxy-2-propyl)benzene or a concentrated liquid thereof is cooled in crystallization tank while being stirred so that bis(2-hydroxy-2-propyl)benzene is selectively deposited. The cooling temperature is, but not limited to, not more than 25°C, for example.

The filtering operation is an operation of separating bis(2-hydroxy-2-propyl)benzene from the organic solvent. The filtering operation is, for example, depressurized filtration, pressurized filtration, or centrifugal filtration. Among these filtrations, the filtering operation is more preferably centrifugal filtration from the viewpoint of operability.

The present invention is not limited to the embodiments, but can be altered by a skilled person in the art within the scope of the claims. The present invention also encompasses, in its technical scope, any embodiment derived by combining technical means disclosed in differing embodiments.

[Examples]

The present invention will be described below in more detail through Examples and Comparative Examples. However, the present invention is not limited to the Examples below.

[Synthesis Example 1]

An oxidation reaction of meta-diisopropylbenzene by the air was carried out to provide an oxidized oil containing 1-(hydroperoxyisopropyl)-3-(hydroxyisopropyl)benzene (which, hereinafter, can be referred to as CHPO) and 1,3-bis(hydroperoxyisopropyl)benzene (which, hereinafter, can be referred to as DHPO). The resultant oxidized oil underwent an extracting operation through 7 mass% aqueous sodium hydroxide solution, and then underwent an extracting operation through methyl isobutyl ketone so that a methyl isobutyl ketone solution (solution I) containing CHPO and DHPO was obtained.

Hydrogen and solution I were continuously supplied to a reactor that included an alumina-supported palladium catalyst serving as a catalyst, so that CHPO and DHPO in the solution I and hydrogen were caused to react together. A resultant liquid was caused to pass through a metal filter, so that the alumina-supported transition metal catalyst was separated. A reaction liquid containing 1,3-bis(2-hydroxy-2-propyl)benzene and methyl isobutyl ketone was thereby obtained.

The obtained reaction liquid underwent a concentrating operation, so that the methyl isobutyl ketone was partially removed. A concentrated liquid was thereby obtained. The obtained concentrated liquid underwent a crystallizing operation and a subsequent separating operation, so that crude 1,3-bis(2-hydroxy-2-propyl)benzene containing methyl isobutyl ketone and water was obtained.

[Examples 1 to 8, Comparative Examples 1 to 4]

By using an evaporator equipped with an oil bath and a vacuum pump, the crude 1,3-bis(2-hydroxy-2-propyl)benzene obtained through Synthesis Example 1 was dried.

Drying was carried out as follows: the crude 1,3-bis(2-hydroxy-2-propyl)benzene was put in a glass flask of the evaporator in an amount of 80 g. The pressure inside the glass flask was set to constant pressure, and the temperature of the oil bath was then increased from room temperature (25°C) to a predetermined temperature, under the constant pressure.

Table 1 shows the pressure inside the glass flask and the predetermined temperature of the oil bath for each of Examples and Comparative Examples. Whether a sublimation phenomenon occurred at the predetermined temperature of the oil bath was checked. Whether a sublimation phenomenon occurred was determined by determining whether solid 1,3-bis(2-hydroxy-2-propyl)benzene was deposited in the upper part of the inner wall of the glass flask. Accordingly, when the deposition of the solid was observed, it was determined that a sublimation phenomenon occurred, and when the deposition of the solid was not observed, it was determined that a sublimation phenomenon did not occur. The results are shown in Table 1.

In Table 1, "No (critical point)" indicates a point at which sublimation started to be observed immediately after the temperature continued to be increased upon a sublimation phenomenon not being observed.

[Table 1]

|  | Temperature (°C) | Pressure (kPaA) | Occurrence of sublimation phenomenon |
|---|---|---|---|
| Example 1 | 60 | 5.3 | No |
| Example 2 | 65 | 10 | No |
| Example 3 | 98 | 50 | No |
| Example 4 | 99 | 60 | No |
| Example 5 | 85 | 5.3 | No (critical point) |
| Example 6 | 89 | 10 | No (critical point) |
| Example 7 | 100 | 40 | No (critical point) |
| Example 8 | 80 | 10 | No |
| Comparative Example 1 | 91 | 5.3 | Yes |
| Comparative Example 2 | 102 | 20 | Yes |
| Comparative Example 3 | 110 | 5.3 | Yes |
| Comparative Example 4 | 120 | 5.3 | Yes |

[Example 9]

By using an evaporator equipped with an oil bath and a vacuum pump, the crude 1,3-bis(2-hydroxy-2-propyl)benzene obtained through Synthesis Example 1 was dried. The crude 1,3-bis(2-hydroxy-2-propyl)benzene before dried included methyl isobutyl ketone (denoted as "MIBK" in Table 2 and also in Table 3) in a concentration of 2.9 mass%.

Drying was carried out as follows: the crude 1,3-bis(2-hydroxy-2-propyl)benzene (1,3-bis(2-hydroxy-2-propyl)benzene is denoted as "MDO" in Table 2 and also in Table 3) obtained through Synthesis Example 1 was put in the glass flask of the evaporator in an amount of 80 g. The pressure inside the glass flask and the predetermined temperature of the oil bath were the same as those in Example 2 and operations that were the same as those in Examples 1 to 4 were carried out. Whether a sublimation phenomenon occurred was determined in the same manner as in Examples 1 to 4. No sublimation phenomenon was found to occur.

After the drying, 75 g of 1,3-bis(2-hydroxy-2-propyl)benzene (white solid) was obtained. The obtained white solid contained methyl isobutyl ketone in a concentration of 0.03 mass%. The results are shown in Table 2.

[Table 2]

| | Tempe-rature (°C) | Pressure (kPaA) | Occurrence of sublimation phenomenon | MDO (g) | | MIBK concentration (wt%) | |
|---|---|---|---|---|---|---|---|
| | | | | Before dried | After dried | Before dried | After dried |
| Example 9 | 65 | 10 | No | 80 | 75 | 2.9 | 0.03 |

[Example 10]

The same operations as in Synthesis Example 1 were carried out, so that crude 1,3-bis(2-hydroxy-2-propyl)benzene containing methyl isobutyl ketone and water was obtained.

The obtained crude 1,3-bis(2-hydroxy-2-propyl)benzene was put in a dryer in an amount of 100 parts by mass. The temperature inside the dryer was then increased from 54°C to 80°C under a pressure of 5.3 kPaA, and drying was additionally carried out for four hours at 80°C.

After the drying, 97 parts by mass of 1,3-bis(2-hydroxy-2-propyl)benzene (white solid) was obtained. The 1,3-bis(2-hydroxy-2-propyl)benzene after dried contained methyl isobutyl ketone in a concentration of 0.004 mass% and contained water in a concentration of 0.01 mass%.

[Example 11]

The same operations as in Synthesis Example 1 were carried out, so that crude 1,3-bis(2-hydroxy-2-propyl)benzene containing methyl isobutyl ketone and water was obtained.

The obtained crude 1,3-bis(2-hydroxy-2-propyl)benzene was put in a dryer in an amount of 100 parts by mass. The temperature inside the dryer was then increased from 56°C to 80°C under a pressure of 5.3 kPaA, and drying was additionally carried out for four hours at 80°C.

After the drying, 98 parts by mass of 1,3-bis(2-hydroxy-2-propyl)benzene (white solid) was obtained. The 1,3-bis(2-hydroxy-2-propyl)benzene after dried contained methyl isobutyl ketone in a concentration of 0.02 mass% and contained water in a concentration of 0.01 mass%.

[Table 3]

| | Temperature (°C) | Pressure (kPaA) | Occurrence of sublimation phenomenon | MDO (parts by mass) | | Concentration after dried (wt%) | |
|---|---|---|---|---|---|---|---|
| | | | | Before dried | After dried | MIBK | Water |
| Example 10 | 80 | 5.3 | No | 100 | 97 | 0.004 | 0.01 |
| Example 11 | 80 | 5.3 | No | 100 | 98 | 0.02 | 0.01 |

(Summation)

As shown in Table 1, when the crude aqueous bis(2-hydroxy-2-propyl)benzene was dried under the condition in which bis(2-hydroxy-2-propyl)benzene does not sublime and which lies below a vapor pressure curve of water, solid 1,3-bis(2-hydroxy-2-propyl)benzene was not deposited in the upper part of the inner wall of the glass flask. As is clear from Tables 2 and 3, drying under this condition enables both prevention of deposition of solid 1,3-bis(2-hydroxy-2-propyl)benzene in the upper part of the inner wall of the glass flask and efficient removal of water and the organic solvent.

Industrial Applicability

The production method in accordance with an embodiment of the present invention yields an effect of preventing blockage in a pipe in a step of drying (2-hydroxy-2-propyl)benzene and thereby enabling enhancement of the production efficiency. The present invention is therefore useful in the fields involving production of bis(2-hydroxy-2-propyl)benzene.

**Claims**

1. A method for producing bis(2-hydroxy-2-propyl)benzene including a drying step of drying aqueous bis(2-hydroxy-2-propyl)benzene containing at least water, the method comprising
a drying step of drying the aqueous bis(2-hydroxy-2-propyl)benzene under a condition in which bis(2-hydroxy-2-propyl)benzene does not sublime and which lies below a vapor pressure curve of water.

2. The method according to claim 1, wherein

in the drying step, the aqueous bis(2-hydroxy-2-propyl)benzene is dried at a temperature and a pressure that satisfy Expression (1) below and that lie below a vapor pressure curve of water:

$$P \geq 4 \times 10^{-21} \times T^{11} \quad (1),$$

where T is temperature (in °C) and P is pressure (in kPaA).

3. The method according to claim 1 or 2, wherein
the aqueous bis(2-hydroxy-2-propyl)benzene is at least one selected from 1,3-(2-hydroxy-2-propyl)benzene and 1,4-(2-hydroxy-2-propyl)benzene.

4. The method according to any one of claims 1 to 3, wherein
the aqueous bis(2-hydroxy-2-propyl)benzene further contains an organic solvent.

5. The method according to claim 4, wherein
the organic solvent is at least one selected from diisopropylbenzene, methyl isobutyl ketone, phenethyl ether, diisopropyl ether, benzyl alcohol, and phenylethanol.

## FIG. 1

## INTERNATIONAL SEARCH REPORT

| International application No. |
|---|
| PCT/JP2020/045843 |

**A.    CLASSIFICATION OF SUBJECT MATTER**
Int. Cl. C07C29/76(2006.01)i, C07C33/26(2006.01)i
FI: C07C29/76, C07C33/26

According to International Patent Classification (IPC) or to both national classification and IPC

**B.    FIELDS SEARCHED**

Minimum documentation searched (classification system followed by classification symbols)
Int. Cl. C07C29/76, C07C33/26

Documentation searched other than minimum documentation to the extent that such documents are included in the fields searched
Published examined utility model applications of Japan     1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)
JSTPlus/JMEDPlus/JST7580 (JDreamIII); CAplus/REGISTRY (STN)

**C.    DOCUMENTS CONSIDERED TO BE RELEVANT**

| Category* | Citation of document, with indication, where appropriate, of the relevant passages | Relevant to claim No. |
|---|---|---|
| X | JP 2007-246512 A (SUMITOMO CHEMICAL CO., LTD.) 27 September 2007, claims, examples, paragraph [0038] | 1-5 |
| A | II 基礎的化学技術−4 章 化学合成技術．化学便覧．Applied Chemistry Ed. 6th Ed., 2003, p. 176, 4.3.1.a, non-official translation (II: Basic Chemical Technology – Chapter 4: Chemical Synthesis Technologies. Handbook of Chemistry.) | 1-5 |
| A | 7 平衡蒸気圧の測定．新実験化学講座 2 基礎技術 1 熱・圧力．1977, p. 336, 7.1.3, non-official translation (7: Measurement of Equilibrium Vapor Pressure. New Experimental Chemistry Course 2: Basic Techniques 1 – Heat and Pressure.) | 1-5 |

☐  Further documents are listed in the continuation of Box C.          ☒  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 20.01.2021 | 02.02.2021 |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

Information on patent family members

International application No.

PCT/JP2020/045843

| Patent Documents referred to in the Report | Publication Date | Patent Family | Publication Date |
|---|---|---|---|
| JP 2007-246512 A | 27.09.2007 | US 2007/0197838 A1 claims, examples, paragraph [0052] | |

Form PCT/ISA/210 (patent family annex) (January 2015)

**EP 4 089 067 A1**

**Patent documents cited in the description**

- JP 2007246512 A